# EUROPEAN PATENT SPECIFICATION

(11) **EP 4 312 926 B1**
(45) Date of publication and mention of the grant of the patent: **26.03.2025**
(21) Application number: 22724135.3
(22) Date of filing: 04.05.2022
(51) Int. Cl.: A61F 13/0246, A61F 13/05

(54) **DRESSING WITH SELECTIVE VIEWING ACCESS AND RECLOSURE SYSTEM**
VERBAND MIT SELEKTIVEM SICHTZUGANGS- UND WIEDERVERSCHLUSSSYSTEM
PANSEMENT AVEC ACCÈS SÉLECTIF À LA VISUALISATION ET SYSTÈME DE REFERMETURE

(30) Priority: 06.05.2021 US 202163185061 P
(43) Date of publication of application: 07.02.2024
(73) Proprietor: KCI Manufacturing Unlimited Company, Athlone, Co. Westmeath, N37XF22 (IE)
(72) Inventor: REHBEIN, Jonathan G., San Antonio, Texas 78265 (US); CARROLL, Christopher J., San Antonio, Texas 78265 (US)
(74) Representative: Simmons & Simmons
(86) International application number: PCT/IB2022/054108
(87) International publication number: WO 2022/234471

(56) References cited:
- EP-A1- 2 422 757
- WO-A1-2020/159859
- US-A- 5 086 763
- US-A- 6 124 520

## Description

### BACKGROUND

The present disclosure relates generally to wound dressings. More specifically, the present disclosure relates to wound dressings for negative pressure wound therapy systems.

NPWT is a therapeutic technique used to promote healing in acute or chronic tissue wounds. NPWT systems are configured to apply a negative pressure to a wound, through a dressing that is connected to the wound (e.g., to the skin surrounding the wound). The negative pressure may be utilized to remove infectious materials, and to help promote wound closure and healing. In order to maintain adequate pressure at the wound site, the dressing must form an airtight seal with the patient's tissue. In some instances, it may be desirable to remove the dressing from the wound during treatment to inspect the state of the wound and assess healing progress. This process requires removal of the dressing from the patient, which may degrade the adhesive used to secure the dressing and renders the dressing unusable. It would be desirable to provide a device that allows for inspection of the wound without sacrificing the dressing.

### SUMMARY

The invention is defined by the independent claim appended hereto. A selection of optional features of the invention is set out in the dependent claims. WO202/159859 discloses relevant technical background.

### BRIEF DESCRIPTION OF THE FIGURES

FIG. 1 is a perspective view of a dressing for a negative pressure wound therapy system, according to an illustrative embodiment.
FIG. 2 is an exploded view of the dressing of FIG. 1.
FIG. 3 is a perspective view of a touch fastener for the dressing of FIG. 1.
FIGS. 4A-4C are side views of a touch fastener in various stages of assembly, according to an illustrative embodiment.
FIG. 5 is a perspective view of a dressing for a negative pressure wound therapy system under an applied pressure, according to an illustrative embodiment.
FIG. 6 is a perspective view of the dressing of FIG. 5 after removing an upper cover from a patient interface layer.
FIG. 7 is a perspective view of the dressing of FIG. 5 during reattachment of the upper cover to the patient interface layer.
FIG. 8 is a perspective view of the dressing of FIG. 5 under an applied pressure after reattaching the upper cover to the patient interface layer.
FIG. 9 is a flow diagram of a method of making a resealable and reclosable dressing, according to an illustrative embodiment.

### DETAILED DESCRIPTION

### Overview

Referring generally to the Figures, a dressing with a removable and resealable reclosure system for a negative pressure wound therapy (NPWT) system is shown. The dressing is structured to engage the NPWT system with a patient's skin or other tissue and to provide a substantially airtight seal in an area surrounding the wound. According to an illustrative embodiment, the dressing is a multi-layer dressing that includes a patient interface layer that is structured to bond to the tissue surrounding the wound area, and an upper cover that is applied over the patient interface layer to centrally over the wound site 10 and covers the wound site 10. In other embodiments, the dressing 100 may only cover a portion of the wound site 10 (e.g., a single leg of an incision, a first portion of an irregularly shaped wound, etc.).

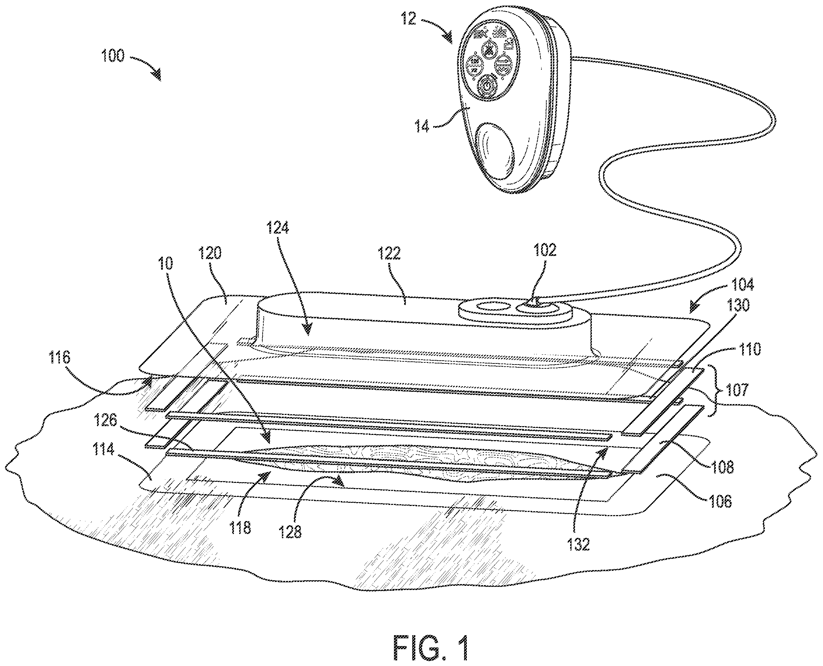

According to an illustrative embodiment, the dressing 100 is used with and/or is part of an NPWT system 12. As shown in FIG. 1, the closure dressing 100 includes a track pad 102 and tubing that is structured to fluidly couple the dressing 100 to a negative pressure source 14 (e.g., a vacuum pump, etc.) to communicate negative pressure to the wound site 10 through the closure dressing 100. The track pad 102 is coupled to an upper cover 104 of the dressing 100, at an intermediate position (e.g., central position, etc.) along the upper cover 104. The track pad 102 includes an airtight connection mechanism that fluidly connects a patient facing side of the upper cover 104 to the tubing. The NPWT system 12 may further include a canister, disposed between the negative pressure source 14 and the track pad 102 to capture any fluid (e.g., wound exudate, antiseptic, instillation fluid, etc.) that is pulled away from the wound site 10. In other embodiments, the dressing 100 may be used independently from the NPWT system 12, as a standalone wound dressing.

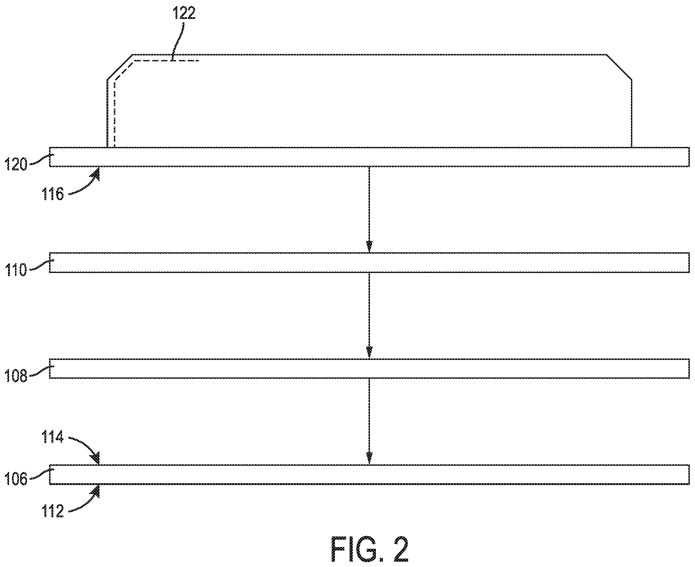

As shown in FIG. 1, the closure dressing 100 includes a patient interface layer 106, a touch fastener 107, and the upper cover 104. The touch fastener 107 includes a first fastening layer 108 and a second fastening layer 110. In other embodiments, the dressing 100 may include additional, fewer, and/or different layers. FIG. 2 shows an exploded view of the dressing 100. As shown in FIGS. 1-2, an outer surface 112 (e.g., wound facing surface, etc.) the patient interface layer 106 is bonded (e.g., adhered) or otherwise coupled to the wound site 10 (e.g., the periwound tissue, skin surrounding the wound, etc.). An inner surface 114 of the patient interface layer 106 is bonded or otherwise coupled to first fastening layer 108. The patient interface layer 106 may be permanently affixed to the first fastening layer 108 such that the first fastening layer 108 cannot be removed without damaging the patient interface layer 106. For example, the first fastening layer 108 may be bonded to the patient interface layer 106 using glue, epoxy, and/or another suitable adhesive product (e.g., a pressure sensitive adhesive, etc.). In another embodiment, the first fastening layer 108 may be laminated to the patient interface layer 106. Similarly, the second fastening layer 110 is bonded or otherwise coupled to a first side 116 (e.g., inner side, inner surface, wound-facing surface, etc.) of the upper cover 104 and may be permanently affixed to the upper cover 104.

As shown in FIGS. 1-2, the first fastening layer 108 and the second fastening layer 110 are "sandwiched" or otherwise disposed in between the patient interface layer 106 and the upper cover 104, or other components of the dressing. As shown in FIG. 1, the first fastening layer 108 and the second fastening layer 110 extend along all the sides of the dressing 100 and extend along a perimeter of an opening 118 in the patient interface layer 106. In the embodiment of FIG. 1, the first fastening layer 108 and the second fastening layer 110 circumscribe the opening 118 in the patient interface layer 106. In other embodiments, the first fastening layer 108 and the second fastening layer 110 are disposed along only a portion of the perimeter of the opening 118 (e.g., along only two sides of the closure dressing 100, three sides, etc.).

### Patient Interface Laver

The patient interface layer 106 is configured to engage with a patient's skin or tissue in the area surrounding the wound and to form a substantially airtight seal between the wound site 10 and the external environment. As shown in FIG. 1, the outer surface 112 of the patient interface layer 106 is bonded to and sealingly engaged with the wound site 10. The inner surface 114 of the patient interface layer 106 is coupled to the first side 116 of the upper cover. 104. The patient interface layer 106 may be made from a polymer film (e.g., a polyurethane film), a medical textile (e.g., Asahi nylon), and/or another suitable material. The patient interface layer 106 may include a suitable low tack adhesive (e.g., silicone or polyurethane gel) to facilitate bonding with the tissue at the wound site 10 (or surrounding the wound site 10). The adhesive may be applied to the outer surface 112 of the patient interface layer 106 proximate to an outer perimeter of the patient interface layer 106, and/or at any other suitable location along the outer surface 112. In some embodiments, the adhesive may be distributed evenly across the outer surface 112. In other embodiments, the patient interface layer 106 is a double-sided adhesive layer that includes a pressure sensitive adhesive on both the inner surface 114 and the outer surface 112.

In some embodiments, the patient interface layer 106 may be substantially the same shape and have the same area as the upper cover 104. As shown in FIG. 1, the patient interface layer 106 defines an opening 118 that is disposed centrally along the patient interface layer 106 (e.g., a central position, in the middle of the patient interface layer 106, etc.). The opening 118 provides fluid communication between the wound site 10 (e.g., wound) and an absorbent material in the upper cover 104. In other embodiments, the patient interface layer 106 includes a plurality of openings extending across the patient interface layer 10. It will be appreciated that the size, shape, and number of openings may be different in various alternative embodiments.

### Upper Cover

The upper cover 104 (e.g., top cover, etc.) substantially encloses the area above the wound site 10 and provides a fluid connection between the NPWT system (e.g., vacuum pump) and the wound site 10. As shown in FIGS. 1-2, the upper cover 104 includes a drape 120, a track pad 102 (e.g., a negative pressure interface, etc.), and an absorbent material 122. The drape 120 defines a recessed area 124 (e.g., pocket, cavity, etc.) at a central position along the drape 120 that is sized to receive the absorbent material 122 therein. The track pad 102 is coupled to the drape 120 at the recessed area 124 and provides the fluid connection between the drape 120 and the tubing for the NPWT system. The drape 120 may be a Tagaderm^{™} layer (e.g., a transparent or partially transparent medical dressing) made from a thin high moisture vapor transmission rate (MVTR) adhesive coated polyurethane film such as Inspire 2327/2317 or another polyurethane or polyethylene film. The drape 120 may be at least partially coated in adhesive, for example, on the wound-facing side of the drape 120 (e.g., the first side 116 of the upper cover 104), in order to bond the drape 120 to the second fastening layer 110 and/or the absorbent material 122.

As shown in FIG. 1, the absorbent material 122 (e.g., layer, sheet, etc.) is disposed within the recessed area 124 and coupled to the wound facing side of the drape 120. The absorbent material 122 is configured to absorb wound exudate and/or other fluids (e.g., instillation fluids, antiseptics, etc.) and remove them from the wound site 10. In at least one embodiment, the absorbent material 122 is made from a superabsorbent laminate such as may be commercially available from Gelok. In other embodiments, the absorbent material 122 includes a printed absorbent polymer.

In some embodiments, the absorbent material 122 may be formed from or otherwise include a superabsorbent polymer in the form of granules. The superabsorbent polymer may include Luquasorb 1160 or 1161, such as may be commercially available from BASF. The granules may be contained in a water-soluble carrier polymer. One example of the water-soluble carrier polymer is polyvinylpyrrolidone (PVP). The superabsorbent polymer and the water-soluble polymer may be formed into a slurry or a suspension using an organic solvent. The organic solvent may include propanone or propanol and may aid in delivery of the absorbent material 122 to the drape 120 or another carrier (e.g., an absorbent foam manifold disposed within the recessed area 124). In some embodiments, to increase the softness of the superabsorbent granules, a plasticizer may be added to the slurry. In one embodiment, the plasticizer may be water. In some embodiments, the slurry to form the absorbent material 122 may have a formulation of 20 parts by mass of PVP, 10 parts by mass of a superabsorbent polymer, 1 part by mass of glycerol, and 100 parts by mass of propanone. In some embodiments, to plasticize the granules, 1 part to 2 parts by mass of water may be added to the slurry mixture. In other embodiments, a water-soluble polymer superabsorbent precursor, such as acrylic acid or 2-acrylamido-2-methyl-propanesulfonic acid (AMPS), with suitable UV curing additives, may replace the superabsorbent polymer. Such a precursor may be a relatively low viscosity solution and can be printed onto at least one of the drape 120 or a separate carrier and exposed to UV light to form a soft gel, eliminating the need for a plasticizer. In some embodiments, the water-soluble polymer superabsorbent precursor may be similar to that used for preparing hydrogel coatings.

By way of example, the slurry mixture may be applied to the wound-facing side of the drape 120 to form an absorbent layer. In some embodiments, the slurry may be applied to the drape 120 through standard printing methods, such as silk screen printing, gravure printing, or by x-y plotter printing. The absorbent layer may be applied in a variety of different shapes such as circles, squares, hexagons, hoops/halos, stars, crosses, a range of lines, or any combination of shapes. The absorbent layer may be substantially evenly distributed on the drape 120 within the recessed area 124. In some embodiments, the absorbent material 122 may include a flexible plasticized hydrophilic polymer matrix having a substantially continuous internal structure. In some embodiments, the absorbent material 122 may include a combination of different materials.

### Touch Fastener

The touch fastener 107 is disposed between the patient interface layer 106 and the upper cover 104 and is configured to detachably couple the patient interface layer 106 to the upper cover 104. The touch fastener 107 is structured to form a substantially airtight and liquid tight seal between the patient interface layer 106 and the upper cover 104. In the embodiments of FIGS. 1-2, the touch fastener 107 includes a first fastening layer 108 and a second fastening layer 110 that are structured to releasably and resealably couple the upper cover 104 (e.g., drape 120) to the patient interface layer 106.

As shown in FIG. 1, the first fastening layer 108 and the second fastening layer 110 each include a plurality of lineal fastening strips that extend along the sides of the dressing 100 and circumscribe the opening 118 in the patient interface layer 106. The fastening strips of the first fastening layer 108 are substantially aligned with the fastening strips of the second fastening layer 110. In the embodiment of FIG. 1, the first fastening layer 108 and the second fastening layer 110 each includes four fastening strips to encompass all four sides of the opening 118. In other embodiments, the shape, position, and/or number of fastening strips may be different.

In at least one embodiment, first fastening layer 108 and the second fastening layer 110 each include a pair of fastening strips that extend along a longest dimension of the opening 118. For example, as shown in FIG. 1, the first fastening layer 108 may include only two fastening strips, including a first fastening strip 126 extending along a first side 128 of the opening 118, and a second fastening strip 130 positioned on a second side 132 of the opening 118 that is opposite from the first side 128. The first fastening strip 126 and the second fastening strip 130 may be substantially parallel to one another and may be oriented in a direction that is substantially parallel to a longest dimension of the opening 118 and/or a longest dimension of the patient interface layer 106. In this implementation, a thick gel adhesive may be applied to the patient interface layer 106 and/or drape 120, along gaps formed between the ends of the first fastening strip 126 and the second fastening strip 130. The gel adhesive or other bonding agent is configured to be repositionable and replaceable. In other words, the gel adhesive may be tacky and may be pushed to other locations along the patient interface layer 106 and/or pulled away from the patient interface layer 106 without damaging the patient interface layer 106. According to an illustrative embodiment, the gel adhesive is a hydrogel having a water activity within a range between approximately 0.45 and 0.69, a solution uptake within a range between approximately 1000% and 3000%, a tack force within a range between approximately 135 g and 194 g, and a coat weight within a range between approximately, 0.92 kg/m2 and 1.18 kg/m2. In other embodiments, the properties of the hydrogel may be different. In another embodiment, the gel adhesive may include a silicone adhesive, DermaTac^{™} (e.g., a silicone and/or acrylic hybrid drape), and/or a hydrophilic gel adhesive, or another suitably tacky adhesive. Among other benefits, using a gel adhesive or other bonding agent in combination with the fastening strips (e.g., on a short end of the dressing 100) reduces the force required to separate (e.g., peel back, remove, etc.) the upper cover 104 from the patient interface layer 106. The arrangement of fastening strips and gel adhesive may differ in various alternative embodiments. For example, the fastening strips may be used on three sides of the opening 118 in the patient interface layer 106 and the gel adhesive may be using on the fourth, open side.

The touch fastener 107 may be any type of selectively resealable and reclosable mechanical interlock that can provide a substantially airtight and liquid tight seal between the upper cover 104 and the patient interface layer 106. For example, the touch fastener 107 may be a Velcro-style hook and loop fastener that includes (i) a male fastening strip and (ii) a female fastening strip that is structured to couple to the male fastening strip in response to an applied pressure between the male fastening strip and the female fastening strip. For example, in the embodiment of FIGS. 1-2, the second fastening layer 110 may include at least one male fastening strip that is engageable with a female fastening strip of the first fastening layer 108 by pressing the male fastening strip into the female fastening strip, such that at least a portion of the male fastening strip extends into (e.g., protrudes into, is received within, etc.) the female fastening strip.

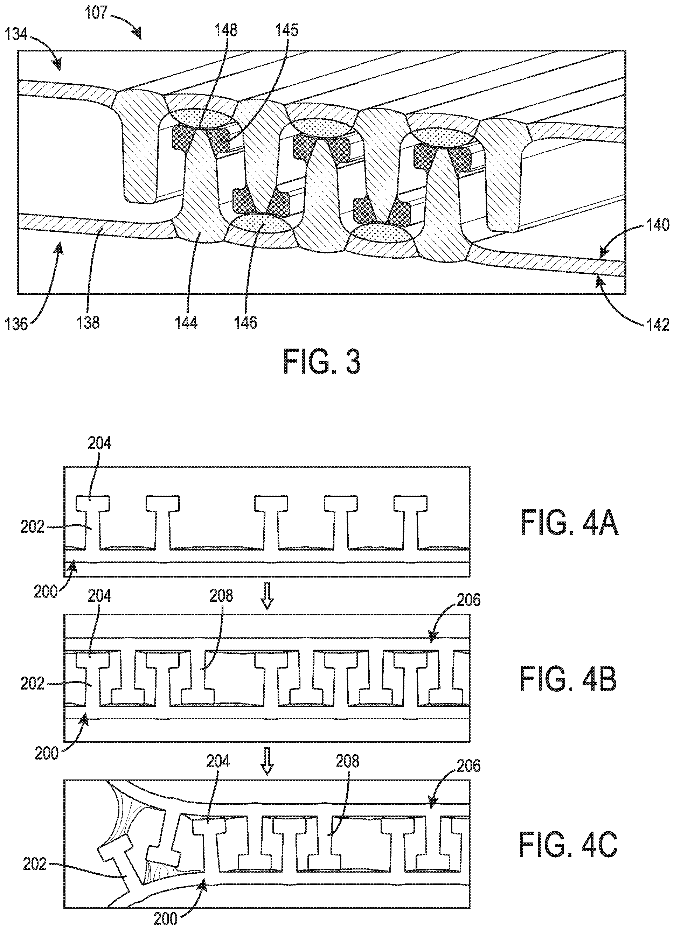

FIG. 3 shows a close up view of the joint that is formed between an upper fastening strip 134 of the first fastening layer 108 and a lower fastening strip 136 of the second fastening layer 110, according to an illustrative embodiment. As shown, upper fastening strip 134 and the lower fastening strip 136 are the same and/or have a substantially similar structure. In other embodiments, the structure of the upper fastening strip 134 and/or the lower fastening strip 136 may be different. In the embodiment of FIG. 3, the lower fastening strip 136 includes a base wall 138 having a first, outer surface 140, and a second, inner surface 142. The lower fastening strip 136 also includes a plurality of stems 144 (e.g., rails, protrusions, etc.) coupled to the base wall 138 along the first surface 140 and extending outwardly from (e.g., away from) the first surface 140 toward the upper fastening strip 134. Each stem 144 extends laterally across the first surface 140, between opposing sides of the lower fastening strip 136. In the embodiment of FIG. 4, the stems 144 are spaced in approximately equal intervals along a longitudinal direction (e.g., perpendicular to the lateral direction) between opposing ends of the lower fastening strip 136. In other embodiments, the number and arrangement of stems 144 may be different.

In some embodiments, the lower fastening strip 136 and/or upper fastening strip 134 include a hook portion (e.g., flange, ledge, etc.) coupled to an outer, free end of each stem 144. For example, as shown in FIG. 3, each stem 144 includes shoulder portions 145 (e.g., shoulders, etc.) extending outwardly from an upper end of the stem 144 at an oblique angle relative to a central axis of the stem 144. The shoulder portions 145 extend away from an upper end of the stem 144 in an at least partially longitudinal direction toward the space between adjacent stems 144. In other embodiments, the shape and/or position of the hook portion of the stem 144 may be different. For example, FIGS. 4A-4C show an example lower fastening strip 200 that includes stems 202 having an outer hook portion 204 that extends in a substantially perpendicular direction relative to the body of each stem 202 (e.g., substantially parallel to the first surface 140). Together, the stem 202 and the hook portion 204 form a substantially "T" shaped protrusion that is structured to engage and interlock with the "T" shaped protrusions of an upper fastening strip 206. In other words, the combination of the stems 202 and hook portion 204 provide a direct mechanical connection between the lower fastening strip 200 and the upper fastening strip 206. In other embodiments, the shape of the hook portion may be different. As shown in FIG. 4B, pressing the upper fastening strip 206 into the lower fastening strip 200 (e.g., against the lower fastening strip 200) engages the stems 202 with the spaces formed between adjacent ones of the stems 208 in the upper fastening strip 206. The hooked portions of the lower fastening strip 200 interlock the hooked portions of the upper fastening strip 206. According to an illustrative embodiment, the stems 202 are formed onto the fastening strips using a micro-profile extrusion process from a flexible plastic material such as polyethylene or another suitable plastic.

In at least one embodiment, the first fastening layer 108 and/or the second fastening layer 110 is a hybrid closure that includes both a non-adhesive-based mechanical fastener (e.g., stems 144) and an adhesive material interposed between adjacent mechanical fastener portions. As shown in FIG. 3, the lower fastening strip 136 and the upper fastening strip 134 each include an adhesive 146 or other bonding agent disposed on the base wall 138, within valleys between adjacent ones of the stems 144. The adhesive 146 is positioned to engage with an outer end surface 148 of a respective one of the stems from an opposing fastening strip, which helps to distribute the adhesive 146 and provides a more robust seal between the upper fastening strip 134 and the lower fastening strip 136. In other embodiments, the adhesive 146 may be applied to different areas of the fastening strips. The adhesive 146 may be a pressure sensitive adhesive material and/or another suitable adhesive product (e.g., silicone, polyurethane gel, etc.).

Among other benefits, the touch fastener 107 structure provides a resealable and reclosable connection between the patient interface layer 106 and the upper cover 104 (see FIG. 1). As shown in FIG. 4C, the touch fastener 207 is structured such that the upper fastening strip 206 can be pulled apart from (e.g., uncoupled from) the lower fastening strip 200 by peeling back one end of the upper fastening strip 206. This causes the stems 202 to pull away from the adhesive material and past the mechanical interlock formed by the hooked portion of the stems 202.

### Dressing Operation

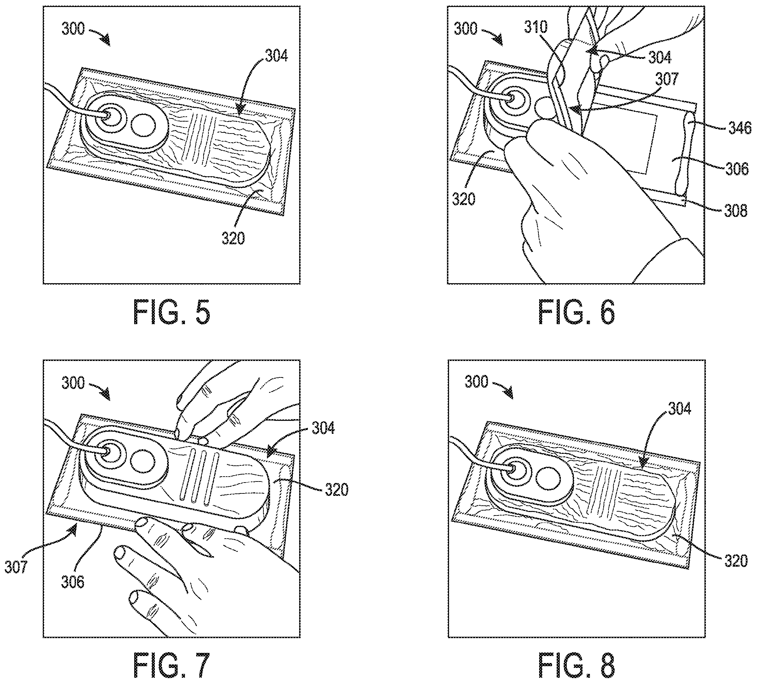

FIGS 5-8 show another example embodiment of a negative pressure wound dressing 300 with a selective viewing access and reclosure system in various states of use. As shown in FIG. 5, the dressing 300 may be provided to a clinician and/or other user as a single unitary structure, with the touch fastener 307 pre-applied in between the upper cover 304 and the patient interface layer 306. Among other benefits, preassembly of the dressing 300 ensures proper alignment between the upper fastening strips and the lower fastening strips, and the most robust connection between adjacent dressing layers.

To apply the dressing 300 to a patient, a clinician or other user simply removes a backing layer from an outer surface of the patient interface layer 306 and applies the exposed surface of the patient interface layer 306 to the tissue. The negative pressure source may then be activated to reduce the pressure in the space above the wound site, in between the wound and the upper cover 304, which compresses the absorbent layer. As shown in FIG. 6, when examination of the wound site is required, the clinician can stop the NPWT therapy (e.g., can deactivate the negative pressure source, decouple the pressure source from the dressing 300, etc.) and begin peeling back the upper cover 304 (e.g., drape 320) from one end of the dressing 300 (e.g., the short end as shown in FIG. 6). In the embodiment shown, the short end of the upper cover 304 is secured to the patient interface layer 306 using a thick gel adhesive product 346. The long ends of the upper cover 304 are secured to the patient interface layer 306 using the touch fastener 307. In other embodiments, the touch fastener 307 may be used without the gel adhesive, by positioning additional sections of the touch fastener 307 to the short ends of the upper cover 304 and patient interface layer 306.

As shown in FIG. 7, after inspecting the wound site, the clinician can reattach the upper cover 304 to the patient interface layer 306 by aligning the fastening strips of both the first fastening layer 308 and the second fastening layer 310. The clinician may then press down on the upper cover 304 to engage the fastening strips of each fastening layer and to thereby engage the mechanical connection of the fastening strips. Finally, the clinician may secure the short end of the upper cover 304 by pressing the short end of the upper cover 304 into the thick gel adhesive on the patient interface layer 306, before restarting the NPWT therapy (e.g., activating the negative pressure source), as shown in FIG. 8.

### Method of Making a Resealable and Reclosable Dressing

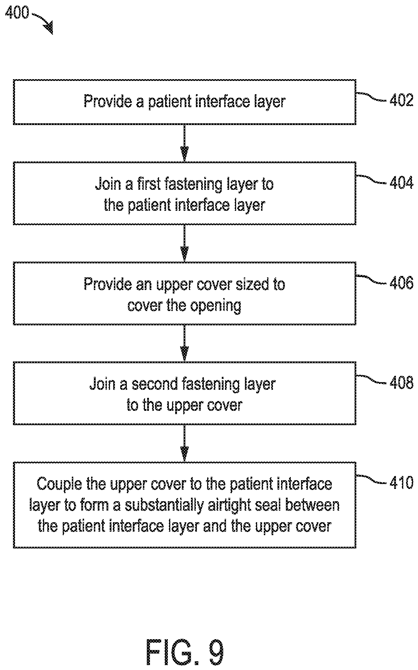

Referring to FIG. 9, a flow diagram of a method 400 of making a resealable and reclosable dressing is shown, according to an illustrative embodiment. The method 400 includes providing a patient interface layer such as a thin film layer, at operation 402. Operation 402 may further includes cutting an opening (e.g., hole) in the patient interface layer, at a central position along the patient interface layer. Operation 402 may further include applying an adhesive product (e.g., a pressure sensitive adhesive) to at least one side of the patient interface layer.

In operation 404, a first fastening layer is joined to the patient interface layer. Operation 404 may include providing the first fastening layer; for example, by providing a plurality of fastening strips, and applying an adhesive product to a backing of the fastening strips and/or to select areas of the patient interface layer (e.g., along a perimeter of the opening). Operation 404 may further include applying the fastening strips of the first fastening layer to the patient interface layer. In at least one embodiment, operation 404 includes applying fastening strips on each side of the opening.

In other embodiments, operation 404 includes applying the fastening strips along only a portion of the perimeter of the opening. For example, operation 404 may include positioning a first fastening strip of the first fastening layer along a direction that is substantially parallel to a longest dimension of the opening and adhering the first fastening strip to the patient interface layer on a first side of the opening. Operation 404 may further include adhering a second fastening strip to a second side of the opening opposite from the first side. Operation 404 may additionally include applying a gel adhesive to the patient interface layer to substantially fill a gap between the first fastening strip and the second fastening strip (e.g., between ends of the first fastening strip and the second fastening strip, such that the fastening strips and gel adhesive together circumscribe the opening).

In operation 406, an upper cover is provided that is sized to cover the opening. Operation 406 may include cutting a drape made from Tagaderm^{™} or another thin film layer to substantially match the size of the patient interface layer. Operation 406 may further include forming a recessed area into the drape using a press operation and/or a heat treating operation. Operation 406 may additionally include placing an absorbent material into the recessed area and/or adhering the absorbent material to the drape using an adhesive material. Operation 406 may also include incorporating a negative pressure coupling (e.g., track pad, etc.) into the drape.

In operation 408, a second fastening layer is joined to the upper cover. Operation 408 may include providing the second fastening layer; for example, by providing a plurality of fastening strips, and applying an adhesive product to a backing of the fastening strips and/or to select areas of the drape that are aligned with the first fastening layer. Operation 408 may further include applying (e.g., adhering) the fastening strips of the second fastening layer to the drape.

In operation 410, the upper cover is coupled to the patient interface layer to form a substantially airtight seal between the upper cover and the patient interface layer. Operation 410 may include aligning the first fastening layer with the second fastening layer and pressing (e.g., via a press or another suitable fixture or process) a male interlocking member of the second fastening layer into a female interlocking member of the first fastening layer (e.g., by engaging the stems, rails, or other mechanical interlock between adjacent fastening strips). In other embodiments, the method 400 may include additional, fewer, and/or different operations.

## Claims

1. A negative pressure wound dressing with a selective viewing access and reclosure system, comprising:
a patient interface layer (106) defining an opening (118) configured for placement about a patient area of treatment;
a top layer (104) sized to cover the opening (118) and comprising a negative pressure interface (102);
a touch fastener (107) disposed between the patient interface layer (106) and the top layer (104), the touch fastener (107) configured to detachably couple the patient interface layer (106) to the top layer (104) and form a substantially airtight seal between the patient interface layer (106) and the top layer (104); and
a negative pressure source (14) coupled to the negative pressure interface (102) on the top layer (104).

2. The negative pressure wound dressing of Claim 1, wherein the touch fastener (107) includes a strip (136, 200) extending along a perimeter portion of the opening (118), the strip comprising a plurality of stems (202) that are configured to mechanically fasten the patient interface layer to the top layer (104) when the top layer (104) is pressed against the patient interface layer (106).

3. The negative pressure wound dressing of Claim 2, wherein the strip (136, 200) further comprises a pressure sensitive adhesive disposed in between adjacent ones of the stems (202).

4. The negative pressure wound dressing of Claim 1, wherein the touch fastener (107) includes both a non-adhesive-based mechanical fastener and an adhesive material.

## Patentansprüche

1. Ein Unterdruckwundverband mit einem selektiven Sichtzugang und Wiederverschließsystem, aufweisend:
eine Patientenberührungsflächenschicht (106), die eine Öffnung (118) definiert, die für eine Platzierung um einen Patientenbehandlungsbereich herum konfiguriert ist;
eine obere Schicht (104), die bemessen ist, um die Öffnung (118) abzudecken, und aufweisend eine Unterdruckberührungsfläche (102);
einen Klettverschluss (107), der zwischen der Patientenberührungsflächenschicht (106) und der oberen Schicht (104) angeordnet ist, wobei der Klettverschluss (107) konfiguriert ist, um die Patientenberührungsflächenschicht (106) mit der oberen Schicht (104) lösbar zu verbinden und eine im Wesentlichen luftdichte Abdichtung zwischen der Patientenberührungsflächenschicht (106) und der oberen Schicht (104) zu bilden; und
eine Unterdruckquelle (14), die mit der Unterdruckberührungsfläche (102) auf der oberen Schicht (104) verbunden ist.

2. Der Unterdruckwundverband nach Anspruch 1, wobei der Klettverschluss (107) einen Streifen (136, 200) einschließt, der sich entlang eines Umfangsabschnitts der Öffnung (118) erstreckt, der Streifen aufweisend eine Mehrzahl von Stegen (202), die konfiguriert sind, um die Patientenberührungsflächenschicht mit der oberen Schicht (104) mechanisch zu verschließen, wenn die obere Schicht (104) gegen die Patientenberührungsflächenschicht (106) gedrückt wird.

3. Der Unterdruckwundverband nach Anspruch 2, wobei der Streifen (136, 200) ferner einen Haftklebstoff aufweist, der zwischen benachbarten der Stege (202) angeordnet ist.

4. Der Unterdruckwundverband nach Anspruch 1, wobei der Klettverschluss (107) sowohl einen mechanischen Verschluss ohne Klebstoffbasis als auch ein Klebstoffmaterial einschließt.

## Revendications

1. Pansement à pression négative doté d'un système sélectif d'accès et de refermeture, comprenant :
une couche d'interface de patient (106) définissant une ouverture (118) conçue pour être placée autour d'une zone de traitement du patient ;
une couche supérieure (104) dimensionnée pour couvrir l'ouverture (118) et comprenant une interface de pression négative (102) ;
une fermeture tactile (107) disposée entre la couche d'interface de patient (106) et la couche supérieure (104), la fermeture tactile (107) étant conçue pour accoupler de manière amovible la couche d'interface de patient (106) à la couche supérieure (104) et former un joint sensiblement étanche à l'air entre la couche d'interface de patient (106) et la couche supérieure (104) ; et
une source de pression négative (14) accouplée à l'interface de pression négative (102) sur la couche supérieure (104).

2. Pansement à pression négative selon la revendication 1, dans lequel l'attache tactile (107) comporte une bande (136, 200) s'étendant le long d'une partie du périmètre de l'ouverture (118), la bande comprenant une pluralité de tiges (202) conçues pour fixer mécaniquement la couche d'interface de patient à la couche supérieure (104), lorsque la couche supérieure (104) est pressée contre la couche d'interface de patient (106).

3. Pansement à pression négative selon la revendication 2, dans lequel la bande (136, 200) comprend en outre un adhésif sensible à la pression disposé entre les tiges (202) adjacentes.

4. Pansement à pression négative selon la revendication 1, dans lequel l'attache tactile (107) comporte à la fois une attache mécanique non adhésive et un matériau adhésif.
